# EUROPÄISCHE PATENTSCHRIFT

(11) **EP 1 055 410 B1**
(45) Veröffentlichungstag und Bekanntmachung des Hinweises auf die Patenterteilung: **22.08.2007**
(21) Anmeldenummer: 00109127.1
(22) Anmeldetag: 05.05.2000
(51) Int. Cl.: A61K 8/19, A61K 8/35, A61K 8/40, A61K 8/49, A61Q 17/04

(54) **Kosmetische oder dermatologische Lichtschutzzubereitungen mit einem Gehalt an Bornitrid und Ethylhexyl-2-cyano-3,3-diphenylacrylat sowie die Verwendung von Bornitrid zur Verstärkung des Lichtschutzfaktors und/oder der UV-Schutzleistung kosmetischer oder dermatologischer Lichtschutzmittel**
Cosmetic or dermatological sunscreen formulations containing boron nitride and ethylhexyl-2-cyano-3,3-diphenylacrylat and use of boron nitride for increasing the light protection factor and /or the UV-A protection of cosmetic or dermatologic light protection agents
Formulations photoprotectrices cosmetiques ou dermatologiques contenant du nitrure de bore et du ethyl-2-cyano-3,3-diphenylacrylat et utilisation de nitrure de bore pour augmenter le facteur photoprotectif et/ou la protection contre le UV-A des compositions cosmétiques ou dermatologiques photoprotectrices

(30) Priorität: 22.05.1999 DE 19923771
(43) Veröffentlichungstag der Anmeldung: 29.11.2000
(73) Patentinhaber: Beiersdorf Aktiengesellschaft, 20245 Hamburg (DE)
(72) Erfinder: Gers-Barlag, Heinrich, Dr., 25495 Kummerfeld (DE); Müller, Anja, 23843 Rümpel (DE)

(56) Entgegenhaltungen:
- EP-A- 0 521 651
- EP-A- 0 685 221
- EP-A- 0 821 941
- EP-A- 0 839 518
- EP-A- 1 013 262
- EP-A- 1 013 263
- WO-A-00/07549
- WO-A-98/46200
- INTROINI C: "NITRURO DI BORO NUOVO INGREDIENTE NEI PRODOTTI PER IL CORPO E NEI SOLARI" COSMETICS AND TOILETRIES. ED. ITALIANA, COSMEO EDITRICE, MILANO, IT, Bd. 18, Nr. 2, 1997, Seiten 71,73,75-76, XP000853904 ISSN: 1121-1377
- DATABASE WPI Section Ch, Week 199424 Derwent Publications Ltd., London, GB; Class A96, AN 1994-197817 XP002238737 ANONYMOUS: "Toiletry and cosmetic compsns. - contg. boron nitride and/or N-lauroyl-lysine." & RESEARCH DISCLOSURE, Bd. 361, Nr. 035, 10. Mai 1994 (1994-05-10), Emsworth, GB

## Beschreibung

Die vorliegende Erfindung betrifft kosmetische und dermatologische Formulierungen, insbesondere kosmetische und dermatologische Lichtschutzformulierungen.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut: Die sogenannte UV-C-Strahlung mit einer Wellenlänge, die kleiner als 290 nm ist, wird von der Ozonschicht in der Erdatmosphäre absorbiert und hat daher keine physiologische Bedeutung. Dagegen verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. Als ein Maximum der Erythemwirksamkeit des Sonnenlichtes wird der engere Bereich um 308 nm angegeben.

Zum Schutz gegen UV-B-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Man hat lange Zeit fälschlicherweise angenommen, daß die langwellige UV-A-Strahlung mit einer Wellenlänge zwischen 320 nm und 400 nm nur eine vernachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u.a. erwiesen, daß selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Femer können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Ferner können bereits sehr geringe Strahlendosen photochemische Reaktionen auslösen. Hierzu gehört insbesondere die Bildung freier Radikale, welche wiederum aufgrund ihrer hohen Reaktivität unkontrollierte Folgereaktionen auslösen können. Um solchen Reaktionen vorzubeugen, können den kosmetischen bzw. dermatologischen Formulierungen zusätzlich Antioxidantien und/oder Radikalfänger zugesetzt werden. So ist beispielsweise vorgeschlagen worden, Vitamin E, eine Substanz mit bekannter antioxidativer Wirkung in Lichtschutzformulierungen einzusetzen, allerdings bleibt hier die erzielte Wirkung weit hinter der erhofften zurück.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z.B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Ein Sonnenbad wird von den meisten Menschen als angenehm empfunden, die nachteiligen Folgen zunächst nicht beachtet. Allerdings hat sich in den letzten Jahren durchaus ein Bewußtsein über die negativen Auswirkungen einer zu intensiven Sonnenbestrahlung herausgebildet, weshalb mehr und stärker schützende Sonnenschutzmittel angewendet werden.

Da die Beiträge der verschiedenen Wellenlängebereiche des UV-Lichtes zu lichtbedingten Hautveränderungen nicht vollständig geklärt sind, geht man heute verstärkt davon aus, daß vorbeugender Schutz sowohl gegen UV-A- als auch gegen UV-B-Strahlen, beispielsweise durch Auftrag von Lichtschutzfiltersubstanzen in Form einer kosmetischen oder dermatologischen Formulierung auf die Haut, von grundsätzlicher Wichtigkeit ist. Kosmetische oder dermatologische Mittel sollen, in dünner Schicht auf die Haut aufgetragen, diese vor den negativen Auswirkungen der Sonnenstrahlung schützen.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen. Je nachdem, in welchem Bereich des UV-Lichtes absorbiert wird, unterscheidet man UV-B-Filter, UV-A-Filter und Breitbandfilter (welche über den gesamten Bereich des UV-A und UV-B eine Filterwirkung zeigen). Durch entsprechende Auswahl des UV-Filters und seiner Konzentration im Sonnenschutzmittel hat man die Möglichkeit, den Grad der Abschirmung des UV-Lichtes zu beeinflussen. Für die Dosierung der Substanzen in den fertigen Formulierungen können die Extinktionswerte allerdings allenfalls eine Orientierungshilfe bieten, denn durch Wechselwirkungen mit Inhaltsstoffen der Formulierung oder der Haut selbst können Unwägbarkeiten auftreten. Ferner ist in der Regel schwierig vorab abzuschätzen, wie gleichmäßig und in welcher Schichtdicke die Filtersubstanz in und auf der Hornschicht der Haut verteilt ist.

Die Wirksamkeit von Sonnenschutzmittel bzw. der ihnen zugrunde liegenden UV-Filter wird in der Regel in biologischen Wirksamkeitsprüfungen unter standardisierten Bedingungen bestimmt.

Der Lichtschutzfaktor (LSF, oft auch SPF (sun protection factor) genannt) gibt die Verlängerung der Sonnenbestrahlung an, die durch Verwendung des Sonnenschutzmittels ermöglicht wird. Er ist der Quotient aus Erythemschwellenzeit mit Sonnenschutzmittel und Erythemschwellenzeit ohne Sonnenschutzmittel.

Zur Prüfung der UV-A-Schutzleistung wird üblicherweise die IPD-Methode verwendet (IPD ≡ immediate pigment darkening). Hierbei wird - ähnlich der Bestimmung des Lichtschutzfaktors - ein Wert ermittelt, der angibt, um wieviel länger die mit dem Lichtschutzmittel geschützte Haut mit UV-A-Strahlung bestrahlt werden kann, bis die gleiche Pigmentierung auftritt wie bei der ungeschützten Haut.

Eine andere, europaweit etablierte Prüfungsmethode ist der Australische Standard AS/NZS 2604:1997. Dabei wird die Absorption der Zubereitung im UV-A-Bereich gemessen. Um den Standard zu erfüllen, muß die Zubereitung mindestens 90 % der UV-A-Strahlung im Bereich 320 -360 nm absorbieren.

Die Einsatzkonzentration bekannter Lichtschutzfiltersubstanzen, insbesondere auch solcher, welche im UV-A-Bereich eine hohe Filterwirkung zeigen, ist häufig gerade in Kombination mit anderen als Festkörper vorliegenden Substanzen begrenzt. Es bereitet daher gewisse formulierungstechnische Schwierigkeiten, höhere Lichtschutzfaktoren bzw. UV-A-Schutzleistung zu erzielen.

Da Lichtschutzfiltersubstanzen in der Regel kostspielig sind und da manche Lichtschutzfiltersubstanzen zudem schwierig in höheren Konzentrationen in kosmetische oder dermatologische Zubereitungen einzuarbeiten sind, war es eine Aufgabe der Erfindung, auf einfache und preiswerte Weise zu Zubereitungen zu gelangen, welche bei ungewöhnlich niedrigen Konzentrationen an herkömmlichen UV-A-Lichtschutzfiltersubstanzen dennoch eine akzeptable oder sogar hohe UV-A-Schutzleistung erreichen.

Eine weitere Aufgabe der Erfindung war es, auf einfache und preiswerte Weise zu Zubereitungen zu gelangen, welche bei ungewöhnlich niedrigen Konzentrationen an herkömmlichen UV-B-Lichtschutzfiltersubstanzen dennoch akzeptable oder sogar hohe LSF-Werte erreichen.

Ferner war Aufgabe der Erfindung durch ungewöhnlich niedrige Konzentrationen an UV-A- und UV-B-Filtersubstanzen und/oder Breitbandfiltern eine dennoch akzeptable oder sogar hohe Breitbandwirkung zu erreichen.

EP-10132262 und WO-00(07549 offenbaren kosmetische Zubereitungen, die übliche UV-Filtersubstanzen und Bornitrid enthalten.
EP-10132263 offenbart eine kosmetische Zubereitung, die BN und Ethylhexyl-2-cyano-3,3-diphenylacrylat enthält.

EP-839518 offenbart kosmetische und dermatologische Zubereitungen, die Bornitrid und UV Filtersubstanzen wie Ethylhexyl-2-cyano-3,3-diphenylacrylat enthalten.

EP-521651 und WO-98/46200 offenbaren kosmetische und dermatologische Zubereitungen, die als anorganische Filtersubstanzen Bornitrid und als organische Filtersubstanzen Ethylhexyl-2-cyano-3,3-diphenylacrylat enthalten können.

C. Introini: 'Nitruro di Boro Nuovo Ingrediente nei Prodotti per il Corpo e nei Solari', COSMETICS AND TOILETRIES. Ed. Italiana, Cosmeo Editrice, Milano, IT, Bd. 18, Nr. 2, 1997, offenbart den Einsatz von Bornitrid in Sonnenschutzzubereitungen und eine gewisse UV-Filterwirkung von Bornitrid. Ethylhexyl-2-cyano-3,3-diphenylacrylat wird nicht genannt.

Research Disclosure, Bd. 361, Nr. 035, 12.Mai 1994 (Derwent AN: 1994-197817) offenbart kosmetische Zubereitungen, die Bornitrid und UV Filtersubstanzen enthalten.

EP-685221 und EP-821941 offenbaren kosmetische Sonnenschutzzubereitungen, die Ethylhexyl-2-cyano-3,3-diphenylacrylat enthalten.

Eine bekannte Lichtschutzfiltersubstanz ist das Ethylhexyl-2-cyano-3,3-diphenylacrylat (Octocrylen), welches von BASF unter der Bezeichnung Uvinul^{®} N 539 erhältlich ist und sich durch folgende Struktur auszeichnet:

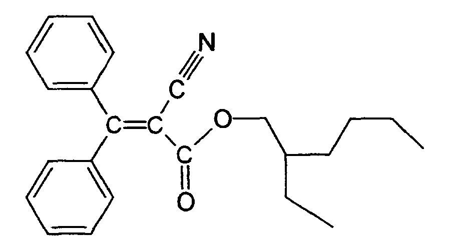

Octocrylen stellt eine hervorragende Lichtschutzfiltersubstanz dar, die weil sie bei Raumtemperatur flüssig ist, einfach in kosmetische Lichtschutzzubereitungen eingearbeitet werden kann und keiner zusätzlichen, die Stabilität der Emulsionen belastenden Ölkomponenten bedarf. Nachteilig ist, daß Octocrylen ein relativ schwacher UV-Absorber ist, so daß in der Regel die Einarbeitung zusätzlicher UV-Filtersubstanzen unumgänglich ist.

Bornitrid (BN oder besser B_{∞}N_{∞}) ist ein homogenes Pulver, welches bekanntermaßen die Eigenschaften kosmetischer Pudergrundlagen verbessert. Im folgenden sind die Strukturen der beiden Hauptmodifikationen des Bornitrids schematisch angegeben, wobei auf die Angabe mesomerer Grenzstrukturen (beim hexagonalen α-Bornitrid) sowie der formalen Ladungen (beim kubischen β-Bornitrid) verzichtet wurde.

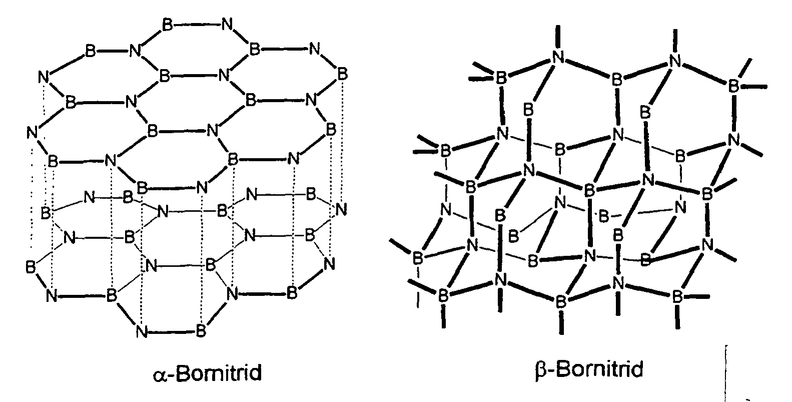

Es war indes überraschend und für den Fachmann nicht vorauszusehen, daß kosmetische oder dermatologische Lichtschutzzubereitungen mit einem Gehalt an α-Bornitrid und Ethylhexyl-2-cyano-3,3-diphenylacrylat, dadurch gekennzeichnet, daß sie 0,1 bis 20 Gew.-% an α-Bornitrid und 0,1 bis 20 Gew.-% an Ethylhexyl-2-cyano-3,3-diphenylacrylat enthalten, wobei die folgende Zusammensetzung (Angaben in Gew.-%) vom Schutzbereich ausgenommen ist:

| | |
|---|---|
| Polyglyceryl-2 Dipolyhydroxystearat | 2 |
| PEG-30 Dipolyhydroxystearat | 2 |
| Caprylic/Capric Triglycerid | 5 |
| Dimethicon | 2 |
| Butylen Glycol Dicaprylat/Caprat | 6 |
| C₁₂₋₁₅- Alkylbenzoat | 6 |
| Vitamen E-Acetat | 0,5 |
| Octyltriazon | 3 |
| Dioctyl Butamido Triazon | 2 |
| Methylbenzyliden Campher | 4 |
| Octocrylen | 10 |
| Bornitrid | 1 |
| Konservierung | 0,5 |
| Glycerin | 5 |
| MgSO₄ | 1 |
| Wasser | ad 100 |

den Nachteilen des Standes der Technik abhelfen würde.

Es war ferner überraschend und für den Fachmann nicht vorauszusehen, dass die Verwendung von Bornitrid zur Steigerung des Lichtschutzfaktors und/oder der UV-A-Schutzleistung kosmetischer oder dermatologischer Lichtschutzzubereitungen, welche 0,1 bis 20 Gew.-% an Bornitrid und 0,1 bis 20 Gew.-% an Ethylhexyl-2-cyano-3,3-diphenylacrylat enthalten, führt.

Das α-Bornitrid stellt die erfindungsgemäß eingesetzte Modifikation dar. Erfindungsgemäß enthalten kosmetische oder dermatologische Lichtschutzzubereitungen 0,1 bis 20 Gew.-%, vorteilhaft 0,5 bis 10 Gew.-%, ganz besonders bevorzugt 1 bis 5 Gew.-% Bornitrid.

Es war erstaunlich, daß Bornitrid im Sinne der vorliegenden Erfindung zu einer Steigerung des Lichtschutzfaktors und/oder der UV-A-Schutzleistung führen würde, da Bornitrid selbst über keine ausgeprägte Absorption im UVA- oder UVB-Bereich verfügt.

Die eingesetzte UV-Schutzfiltersubstanz ist das Ethylhexyl-2-cyano-3,3-diphenylacrylat wobei kosmetische oder dermatologische Lichtschutzzubereitungen erfindungsgemäß 0,1 bis 20 Gew.-%, vorteilhaft 0,5 bis 10 Gew.-%, ganz besonders bevorzugt 1 bis 5 Gew.-% Ethylhexyl-2-cyano-3,3-diphenylacrylat enthalten.

Weitere übliche UV-A-Filtersubstanzen sind Dibenzoylmethanderivate, insbesondere 5-Isopropyldibenzoylmethan (CAS-Nr. 63250-25-9), welches sich durch die Struktur 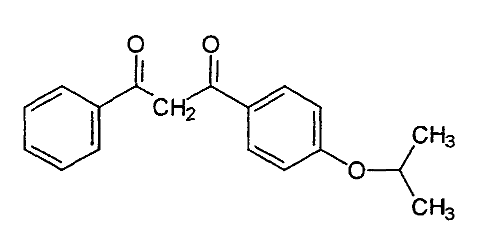 auszeichnet und von Merck unter der Marke Eusolex^{®} 8020 verkauft wird, und/oder das 4-(tert.-Butyl)-4'-methoxydibenzoylmethan (CAS-Nr. 70356-09-1), welches sich durch die Struktur 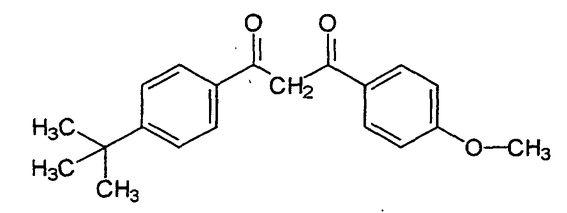 auszeichnet und von Givaudan unter der Marke Parsol^{®} 1789 verkauft wird.

Weitere übliche Lichtschutzfiltersubstanzen weisen das Strukturelement des Methylidencamphers auf, so beispielsweise der 4-Methylbenzylidencampher, welcher sich durch die Struktur 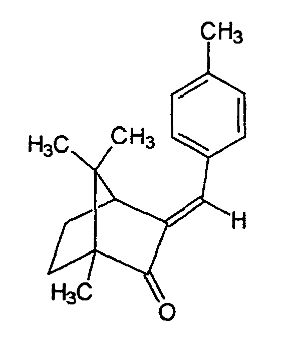 auszeichnet und von Merck unter der Marke Eusolex® 6300 verkauft wird, sowie der Benzylidencampher, welcher sich durch die Struktur 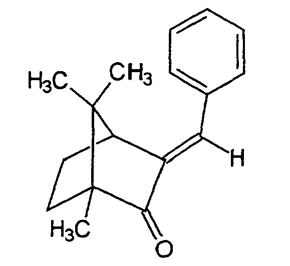 auszeichnet und von der Gesellschaft Induchem unter der Marke Unisol® S22 verkauft wird.

Weitere übliche Lichtschutzfiltersubstanzen weisen das Strukturelement des Benzophenons auf:

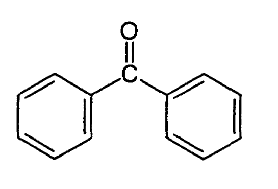

Vorteilhaft können die Substanzen, die das Strukturmotiv des Benzophenons enthalten (in dieser Schrift auch als "Benzophenone" bezeichnet) gewählt werden aus der Gruppe der folgenden Substanzen

| | |
|---|---|
| 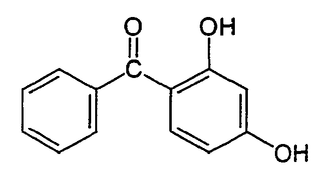 | 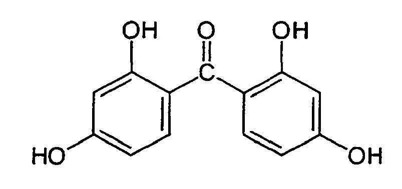 |
| Benzophenon-1 (2,4-Dihydroxybenzophenon) CAS-Nr. 131-56-6 | Benzophenon-2 (2,2',4,4'-Tetrahydroxybenzophenon) CAS-Nr. 131-55-5 |
| 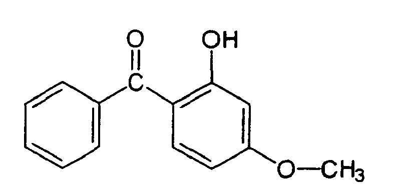 | 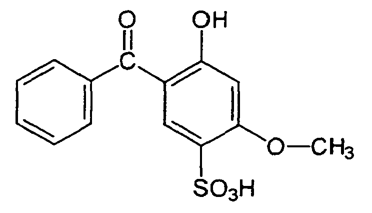 |
| Benzophenon-3 (2-Hydroxy-4-methoxybenzophenon) | Benzophenon-4 (2-Hydroxy-4-methoxybenzophenon-5-sulfonsäure) |
| CAS-Nr. 131-57-7 | CAS-Nr. 4065-45-6 |
| 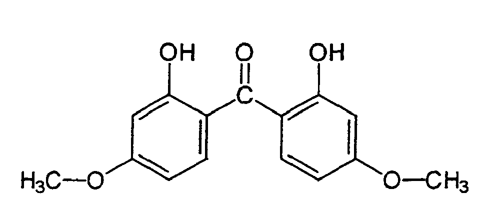 | 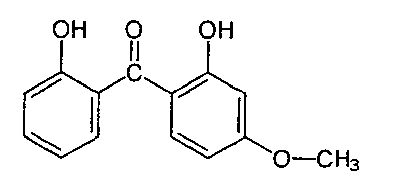 |
| Benzophenon-6 (2,2'-Dihydroxy-4,4'-dimethoxybenzophenon) | Benzophenon-8 (2,2'-Dihydroxy-4-methoxybenzophenon) |
| CAS-Nr. 131-54-4 | CAS-Nr. 131-53-3 |
| 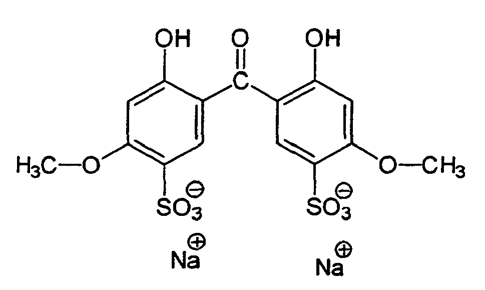 | |
| Benzophenon-9 (Dinatrium-2,2'-dihydroxy-4,4'-dimethoxy-5,5'-disulfobenzophenon) Cas-Nr. 3121-60-6, | |
| ferner Benzophenon-11, CAS-Nr. 1341-54-4. | |

Bevorzugtes Benzophenon ist Benzophenon-3, welches beispielsweise von Merck unter der Warenbezeichnung Eusolex® 4360 verkauft wird.

Von den Dibenzoylmethanderivaten werden vorteilhaft verwendet:

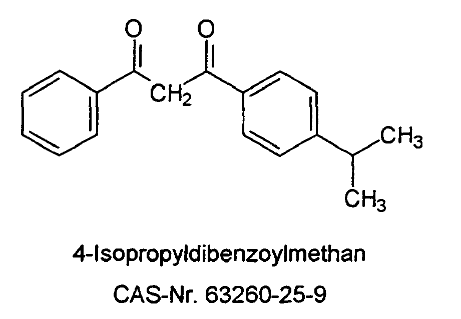

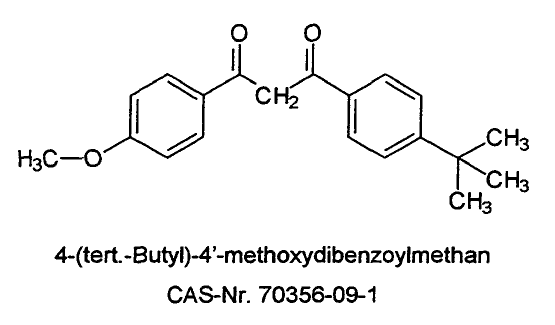

Weitere übliche Lichtschutzfiltersubstanzen sind Salicylsäurederivate wie

| | |
|---|---|
| 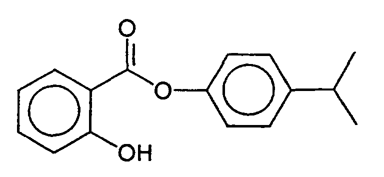 | (4-Isopropylbenzylsalicylat), |
| 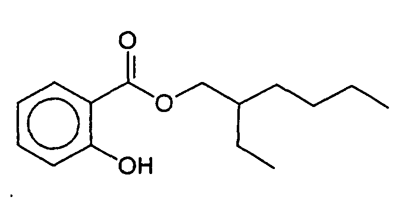 | (2-Ethylhexylsalicylat, Octylsalicylat), |
| 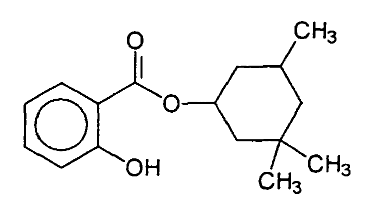 | (Homomenthylsalicylat). |

Weiterere übliche Lichtschutzfiltersubstanzen sind Zimtsäureester, beispielsweise das 2-Ethylhexyl-p-methoxy-cinnamat (4-Methoxyzimtsäure-2'ethylhexylhester), welches von Givaudan unter der Bezeichnung Parsol® MCX erhältlich ist und sich durch folgende Struktur auszeichnet:

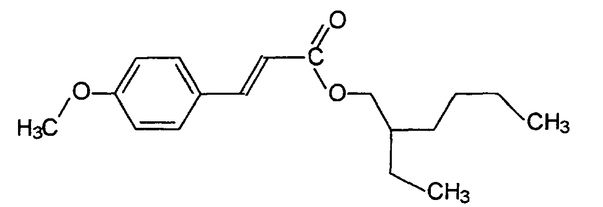

Weitere übliche UV-A-Filtersubstanzen sind die Phenylen-1,4-bis-(2-benzimidazyl)-3,3'-5,5'-tetrasulfonsäure: 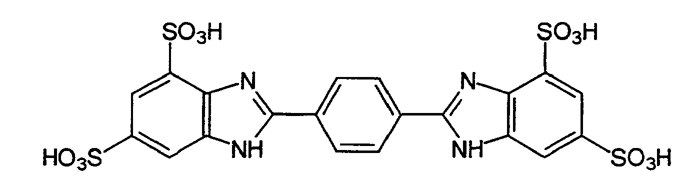 und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz: 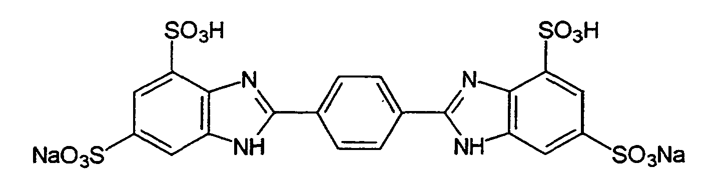 sowie das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

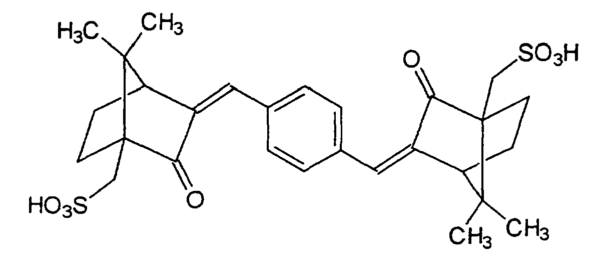

Übliche UV-Filtersubstanzen sind ferner sogenannte Breitbandfilter, d.h. Filtersubstanzen, die sowohl UV-A- als auch UV-B-Strahlung absorbieren.

Breitbandfilter sind beispielsweise Bis-Resorcinyltriazinderivate mit der folgenden Struktur: 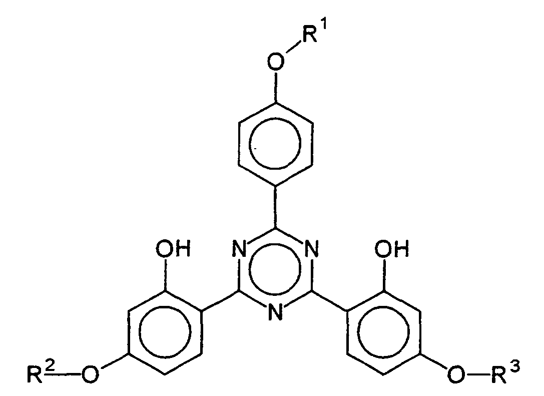 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt ist das 2,4-Bis-{[4-(2-Ethylhexyloxy)-2-hydroxy]-phenyl}-6-(4-methoxyphenyl)-1,3,5-triazin, welches durch folgende Struktur gekennzeichnet ist:

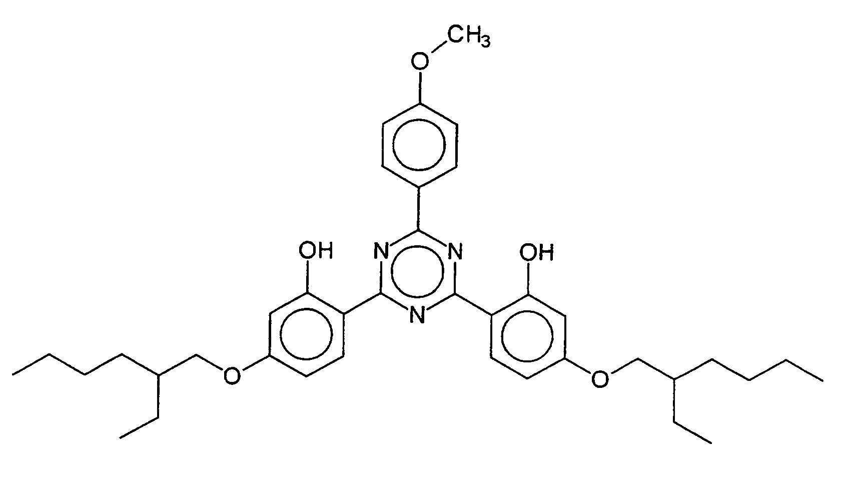

Ein weiterer UV-Filter ist der 4,4',4"-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin.

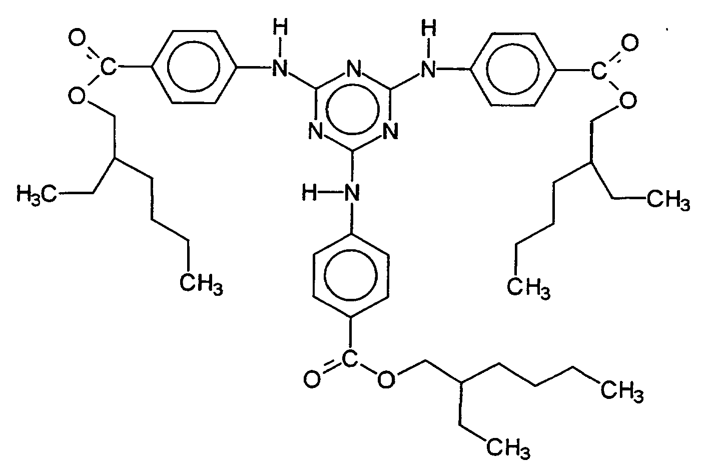

Diese UV-B-Filtersubstanz wird von der BASF Aktiengesellschaft unter der Warenbezeichnung UVINUL® T 150 vertrieben und zeichnet sich durch gute UV-Absorptionseigenschaften aus. Der Hauptnachteil des 4,4',4"-(1,3,5-Triazin-2,4,6-triyltriimino)-tris-benzoësäure-tris(2-ethylhexylesters) ist seine schlechte Löslichkeit in Lipiden. Es bereitete daher in der Vergangenheit gewisse formulierungstechnische Schwierigkeiten, mit Hilfe dieses Filters höhere Lichtschutzfaktoren zu erzielen.

Zwischenzeitlich wurden von verschiedenen Autoren auch andere UV-Filtersubstanzen beschrieben, welche das Strukturmotiv 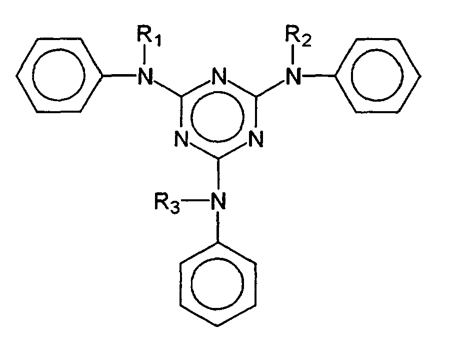 aufweisen und deren Einarbeitung in kosmetische oder dermatologische Lichtschutzformulierungen zumindest gewisse Probleme aufweisen kann. So werden in der Europäischen Offenlegungsschrift 570 838 s-Triazinderivate beschrieben, deren chemische Struktur durch die generische Formel 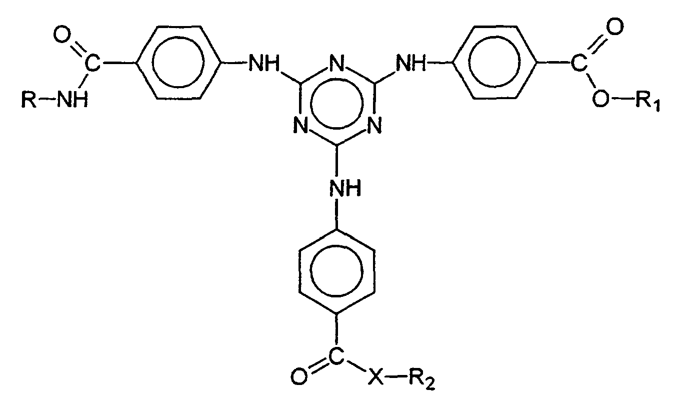 wiedergegeben wird, wobei
- R: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen,
oder ein Wasserstoffatom, ein Alkalimetallatom, eine Ammoniumgruppe oder eine Gruppe der Formel 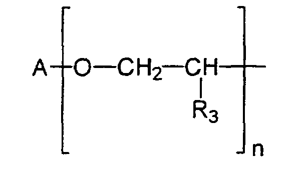 bedeutet, in welcher
- A: einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
- R₃: ein Wasserstoffatom oder eine Methylgruppe darstellt,
- n: eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt, wenn X die NH-Gruppe darstellt, und
einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 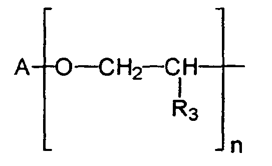 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt, wenn X ein Sauerstoffatom darstellt.

Ein Beispiel für solche unsymmetrisch substituierte s-Triazine stellt das Dioctylbutylamidotriazon dar, dessen chemische Struktur durch die Formel 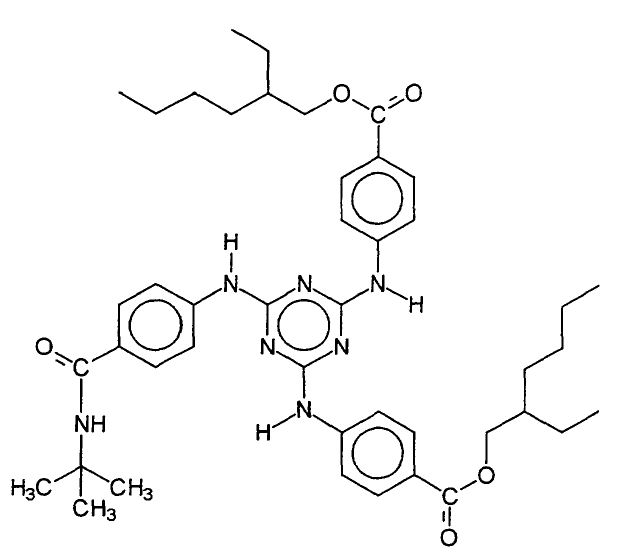 wiedergegeben wird.

Auch andere UV-Filtersubstanzen, deren Einarbeitung in kosmetische oder dermatologische Lichtschutzformulierungen Probleme aufweist, sind bekannt. So werden in der Europäischen Offenlegungsschrift 775 698 Bis-Resorcinyltriazinderivate beschrieben, deren chemische Struktur durch die generische Formel 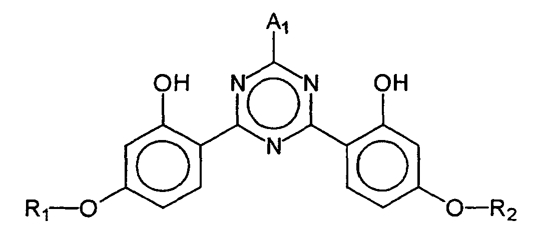 wiedergegeben wird, wobei R₁, R₂ und A₁ verschiedenste organische Reste repräsentieren.

Ferner bekannt ist das 2,4-Bis-{[4-(3-sulfonato)-2-hydroxy-propyloxy)-2-hydroxy]-phenyl}-6-(4-methoxyphenyl)-1,3,5-triazin Natriumsalz, welches durch folgende Struktur gekennzeichnet ist:

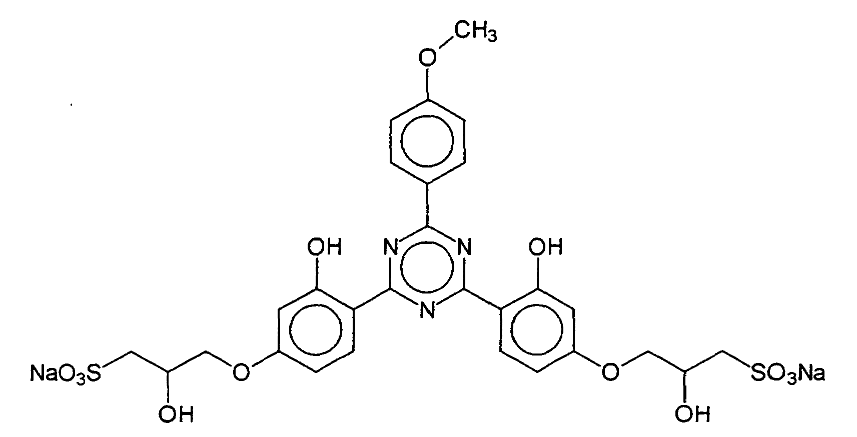

Ferner bekannt ist das 2,4-Bis-{[4-(3-(2-Propyloxy)-2-hydroxy-propyloxy)-2-hydroxy]-phenyl}-6-(4-methoxyphenyl)-1,3,5-triazin, welches durch folgende Struktur gekennzeichnet ist:

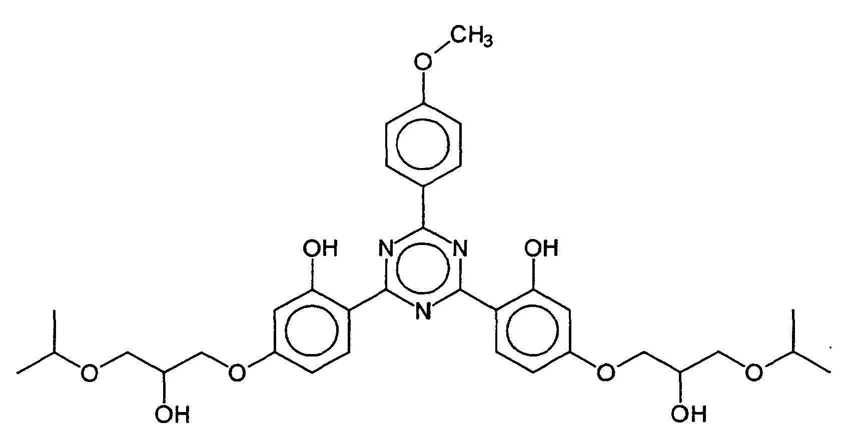

Ferner bekannt ist das 2,4-Bis-{[4-(2-ethyl-hexyloxy)-2-hydroxy]-phenyl}-6-[4-(2-methoxyethyl-carboxyl)-phenylamino]-1,3,5-triazin, welches durch folgende Struktur gekennzeichnet ist:

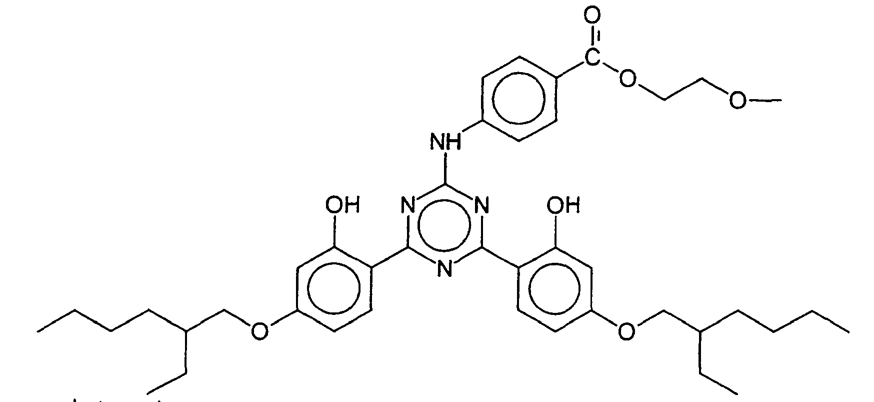

Ferner bekannt ist das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, welches durch folgende Struktur gekennzeichnet ist:

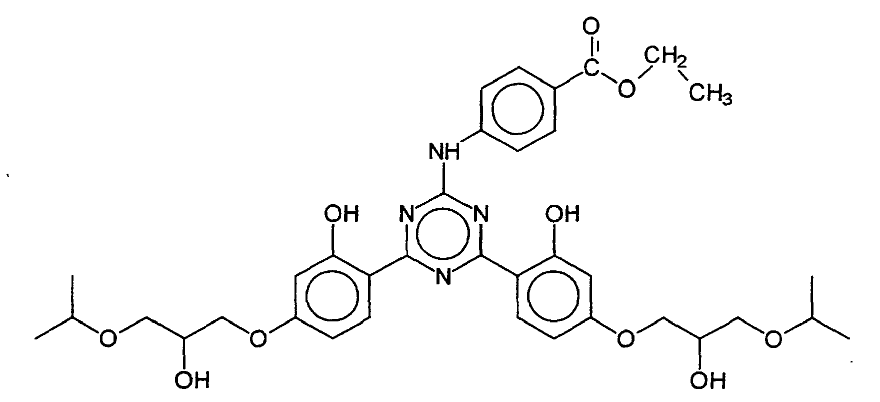

Ferner bekannt ist das 2,4-Bis-{[4-(2-ethyl-hexyloxy)-2-hydroxy]-phenyl}-6-(1-methylpyrrol-2-yl)-1,3,5-triazin, welches durch folgende Struktur gekennzeichnet ist:

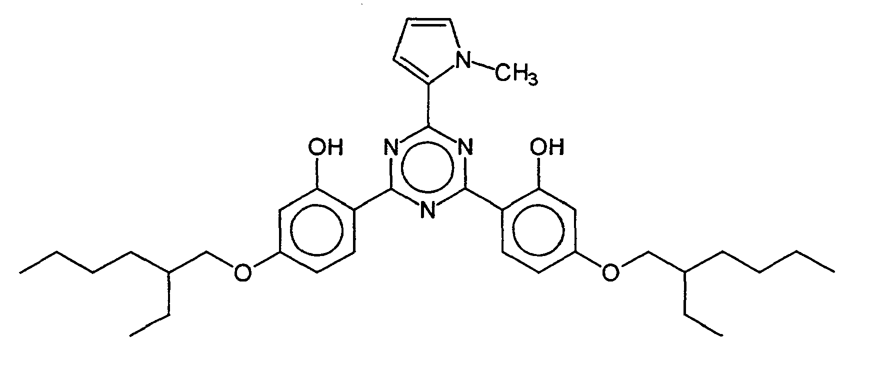

Ferner bekannt ist das 2,4-Bis-{[4-tris(trimethylsiloxy-silylpropyloxy)-2-hydroxy]-phenyl}-6-(4-methoxyphenyl)-1,3,5-triazin, welches durch folgende Struktur gekennzeichnet ist:

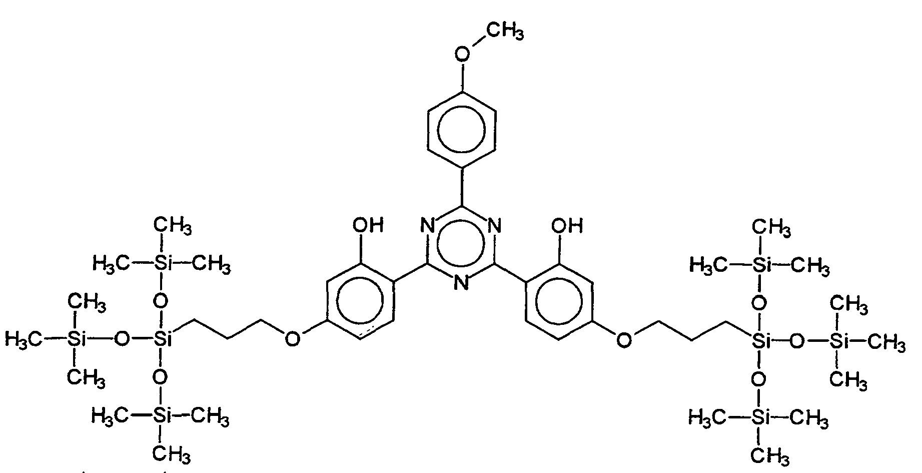

Ferner bekannt ist das 2,4-Bis-{[4-(2"-methylpropenyloxy)-2-hydroxy]-phenyl}-6-(4-methoxyphenyl)-1,3,5-triazin, welches durch folgende Struktur gekennzeichnet ist:

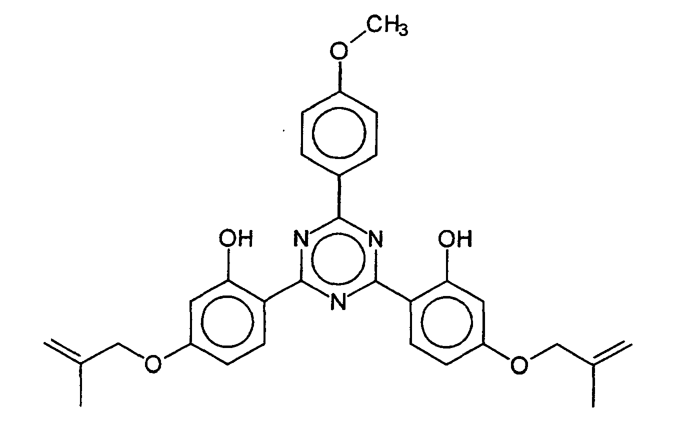

Ferner bekannt ist das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin, welches durch folgende Struktur gekennzeichnet ist:

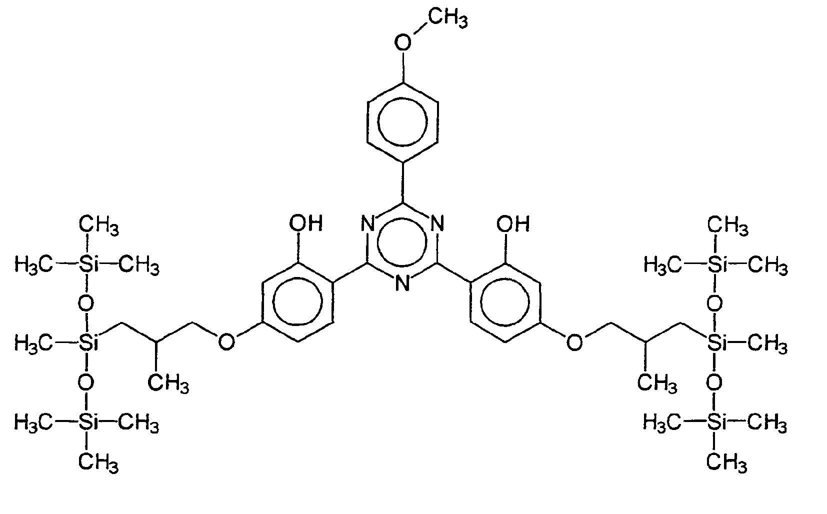

Noch ein weiterer UV-Filter ist das 2,2'-Methylen-bis-(6-(2H-benzotriazol-2-yl)-4-(1,1,3,3-tetramethylbutyl)-phenol), welches durch die chemische Strukturformel 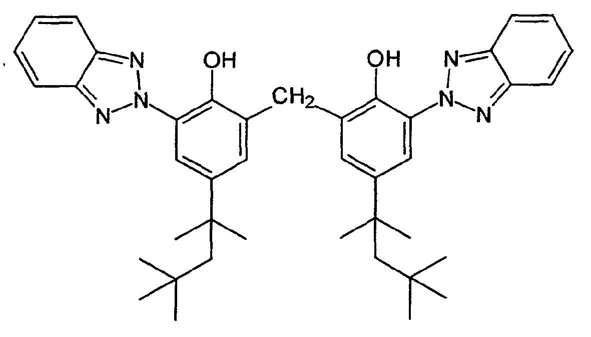 gekennzeichnet ist.

Die UV-B-Filter können öllöslich oder wasserlöslich sein. Vorteilhafte öllösliche UV-B-Filtersubstanzen sind z.B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;

Vorteilhafte wasserlösliche UV-B-Filtersubstanzen sind z.B.:
- Salze der 2-Phenylbenzimidazol-5-sulfonsäure, wie ihr Natrium-, Kalium- oder ihr Triethanolammonium-Salz, sowie die Sulfonsäure selbst;
- Sulfonsäure-Derivate des 3-Benzylidencamphers, wie z.B. 4-(2-Oxo-3-bomylidenmethyl)benzolsulfonsäure, 2-Methyl-5-(2-oxo-3-bornylidenmethyl)sulfonsäure und deren Salze.

Es kann auch von erheblichem Vorteil sein, polymergebundene oder polymere UV-Filter substanzen in Zubereitungen gemäß der vorliegenden Erfindung zu verwenden, insbesondere solche, wie sie in der WO-A-92/20690 beschrieben werden.

Femer kann es gegebenenfalls von Vorteil sein, weitere UV-A- und/oder UV-B-Filtern in kosmetische oder dermatologisch Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Die Liste der genannten UV-Filter, die eingesetzt werden können, soll selbstverständlich nicht limitierend sein.

Die Gesamtmenge an wasserlöslichen UV-Filtersubstanzen in den fertigen kosmetischen oder dermatologischen Zubereitungen, beispielsweise an 2-Phenylbenzimidazol-5-sulfonsäure bzw. deren Salzen und/oder 2-Hydroxy-4-methoxybenzophenon-5-sulfonsäure bzw. deren Salzen und/oder 4-(2-Oxo-3-bornylidenmethyl)benzolsulfonsäure bzw. deren Salzen und/oder 2-Methyl-5-(2-oxo-3-bornylidenmethyl)benzolsulfonsäure bzw. deren Salzen und/oder Benzol-1,4-di(2-oxo-3-bornylidenmethyl)-10-sulfonsäure bzw. deren Salzen, wird vorteilhaft aus dem Bereich von 0,1 - 10,0 Gew.-%, bevorzugt 0,5 - 6,0 Gew.-% gewählt, bezogen auf das Gesamtgewicht der Zubereitungen, falls die Gegenwart dieser Substanzen erwünscht ist.

Die Gesamtmenge an öllöslichen UV-Filtersubstanzen in den fertigen kosmetischen oder dermatologischen Zubereitungen, beispielsweise an 4,4',4''-(1,3,5-Triazin-2,4,6-triyltriimino)-tris-benzoesäure-tris(2-ethylhexylester) und/oder 4-(tert.-Butyl)-4'-methoxydibenzoylmethan und/oder 4-Methylbenzylidencampher, wird vorteilhaft aus dem Bereich von 0,1 - 10,0 Gew.-%, bevorzugt 0,5 - 6,0 Gew.-% gewählt, bezogen auf das Gesamtgewicht der Zubereitungen, falls die Gegenwart dieser Substanzen erwünscht ist.

Die Gesamtmenge an 2-Ethylhexyl-p-methoxy-cinnamat in den fertigen kosmetischen oder dermatologischen Zubereitungen wird, falls die Gegenwart dieser Substanz erwünscht ist, vorteilhaft aus dem Bereich von 0,1 - 15,0 Gew.-%, bevorzugt 0,5 - 7,5 Gew.-% gewählt, bezogen auf das Gesamtgewicht der Zubereitungen.

Die Gesamtmenge an Ethylhexyl-2-cyano-3,3-diphenylacrylat in den fertigen kosmetischen oder dermatologischen Zubereitungen wird vorteilhaft aus dem Bereich von 0,1 - 15,0 Gew.-%, bevorzugt 0,5 - 10,0 Gew.-% gewählt, bezogen auf das Gesamtgewicht der Zubereitungen.

Die Gesamtmenge an einem oder mehreren Salicylsäurederivaten in den fertigen kosmetischen oder dermatologischen Zubereitungen wird vorteilhaft aus dem Bereich von 0,1 - 15,0 Gew.-%, bevorzugt 0,5 - 8,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 - 5,0 Gew.-%, bevorzugt 0,5 - 2,5 Gew.-% zu wählen. Wenn Homomenthylsalicylat gewählt wird, ist es von Vorteil, dessen Gesamtmenge aus dem Bereich von 0,1 - 10,0 Gew.-%, bevorzugt 0,5 - 5,0 Gew.-% zu wählen.

Kosmetische und dermatologische Formulierungen gemäß der Ansprüche können zusätzlich übliche UV-A-, UV-B- und/oder Breitbandfilter in beliebigen Gemischen untereinander, in der Lipidphase und/oder in der wäßrigen Phase enthalten.

Die Gesamtmenge an UV-Filtersubstanzen (UV-A-, UV-B- und/oder Breitbandfilter) in den fertigen kosmetischen oder dermatologischen Zubereitungen wird vorteilhaft aus dem Bereich von 0,1 bis 30 Gew.-%, bevorzugt. 0,1 bis 20,0 Gew.-%, insbesondere 0,5 bis 15,0 Gew.-% gewählt, bezogen auf das Gesamtgewicht der Zubereitungen.

Erfindungsgemäße kosmetische und dermatologische Zubereitungen enthalten ferner vorteilhaft, wenngleich nicht zwingend, anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden. Diese Pigmente sind röntgenamorph oder nicht-röntgenamorph. Besonders bevorzugt handelt es sich um Pigmente auf der Basis von TiO₂.

Röntgenamorphe Oxidpigmente sind Metalloxide oder Halbmetalloxide, welche bei Röntgenbeugungsexperimenten keine oder keine erkenntliche Kristallstruktur erkennen lassen. Oftmals sind solche Pigmente durch Flammenreaktion erhältlich, beispielsweise dadurch, daß ein Metall- oder Halbmetallhalogenid mit Wasserstoff und Luft (oder reinem Sauerstoff) in einer Flamme umgesetzt wird.

In kosmetischen, dermatologischen oder pharmazeutischen Formulierungen werden röntgenamorphe Oxidpigmente als Verdickungs- und Thixotropierungsmittel, Fließhilfsmittel, zur Emulsions- und Dispersionsstabilisierung und als Trägersubstanz (beispielsweise zur Volumenerhöhung von feinteiligen Pulvern oder Pudern) eingesetzt.

Bekannte und in der kosmetischen oder dermatologischen Galenik oftmals verwendete röntgenamorphe Oxidpigmente sind die Siliciumoxide des Typs Aerosil® (CAS-Nr. 7631-86-9. Aerosile®, erhältlich von der Gesellschaft DEGUSSA, zeichnen sich durch geringe Partikelgröße (z.B. zwischen 5 und 40 nm) aus, wobei die Partikel als kugelförmige Teilchen sehr einheitlicher Abmessung anzusehen sind. Makroskopisch sind Aerosile® als lockere, weiße Pulver erkenntlich. Im Sinne der vorliegenden Erfindung sind röntgenamorphe Siliciumdioxidpigmente besonders vorteilhaft, und unter diesen gerade solche des Aerosil®-Typs bevorzugt.

Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974, Aerosil® R976.

Erfindungsgemäß enthalten kosmetische oder dermatologische Lichtschutzzubereitungen 0,1 bis 20 Gew.-%, vorteilhaft 0,5 bis 10 Gew.-%, ganz besonders bevorzugt 1 bis 5 Gew.-% röntgenamorphe Oxidpigmente.

Die nicht-röntgenamorphen anorganischen Pigmente liegen erfindungsgemäß vorteilhaft in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

n TiO₂ + m (RO)₃Si-R'→ n TiO₂ (oberfl.)

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte TiO₂-Pigmente sind beispielsweise unter der Handelsbezeichnung T 805, vorteilhafte TiO₂/Fe₂O₃-Mischoxide unter der Handelsbezeichnung T 817 von der Firma Degussa erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Die erfindungsgemäßen kosmetischen und/oder dermatologischen Formulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen. Entsprechend können die erfindungsgemäßen Zubereitungen, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zubereitungen als Grundlage für pharmazeutische Formulierungen zu verwenden. Bevorzugt sind insbesondere solche kosmetischen und dermatologischen Zubereitungen, die in der Form eines Hautpflege- bzw. Schminkproduktes vorliegen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich zu UV-A-, UV-B- und/oder Breitbandfiltern mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Die jeweils einzusetzenden Mengen an kosmetischen oder dermatologischen Hilfs- und Trägerstoffen und Parfüm können in Abhängigkeit von der Art des jeweiligen Produktes vom Fachmann durch einfaches Ausprobieren leicht ermittelt werden.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 0,1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:
- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die nachfolgenden Beispiele sind nicht erfindungsgemäß Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiel 1

| O/W - Emulsion | Gew.-% |
|---|---|
| Stearinsäure | 1,50 |
| Glycerinmonostearat | 3,00 |
| Caprylsäure/Caprnsäuretriglycerid | 10,00 |
| Dicaprylylether | 5,00 |
| Dimethicon | 2,00 |
| Hydriertes Polyisobuten | 2,00 |
| Vitamen E-Acetat | 0,50 |
| Octocrylen | 10,00 |
| Tinosorb® S | 4,00 |
| Methylbenzylidencampher | 4,00 |
| Titandioxid | 1,00 |
| Konservierung | 0,50 |
| Glycerin | 3,00 |
| Xanthan Gummi | 0,30 |
| Natronlauge 45% | 0,50 |
| Wasser | ad 100,00 |

### Beispiel 2

| O/W - Emulsion | Gew.-% |
|---|---|
| Sorbitanstearat | 3,00 |
| Polyglyceryl-3-methylglucosedistearat | 1,50 |
| Octyldodecanol | 10,00 |
| Dicaprylylether | 5,00 |
| Mineralöl | 5,00 |
| Ricinusöl | 2,00 |
| Butylenglycoldicaprylat/Caproat | 5,00 |
| Vitamen E-Acetat | 0,50 |
| Octocrylen | 8,00 |
| Tinosorb® S | 1,50 |
| Octyltriazon | 4,00 |
| Methylbenzylidencampher | 4,00 |
| Butylmethoxydibenzoylmethan | 3,00 |
| Konservierung | 0,50 |
| Glycerin | 10,00 |
| Xanthan Gummi | 0,20 |
| Pemulen ® TR1 | 0,10 |
| Phenylbenzimidazolsulfonsäure | 2,00 |
| Natronlauge 45% | 1,20 |
| Wasser | ad 100,00 |

### Beispiel 3

| W/O - Emulsion | Gew.-% |
|---|---|
| Polyglyceryl-2-dipolyhydroxystearat | 5,00 |
| Dimethicon | 2,00 |
| Mineralöl | 5,00 |
| Isohexadekan | 5,00 |
| Butylenglycoldicaprylat/Caproat | 5,00 |
| Dioctylbutamidotriazon | 3,00 |
| Octocrylen | 12,00 |
| Tinosorb® S | 3,00 |
| Methylbenzylidencampher | 2,00 |
| Butylmethoxydibenzoylmethan | 2,00 |
| Titandioxid | 4,00 |
| Konservierung | 0,50 |
| Glycerin | 5,00 |
| MgSO₄ | 1,00 |
| Wasser | ad 100,00 |

### Beispiel 4

| W/O - Emulsion | Gew.-% |
|---|---|
| PEG-30-Dipolyhydroxystearat | 4,00 |
| Caprylsäure/Caprnsäuretriglycerid | 5,00 |
| Octyldodecanol | 5,00 |
| Dicaprylylether | 5,00 |
| Mineralöl | 5,00 |
| Hydriertes Polyisobuten | 5,00 |
| Vitamen E-Acetat | 0,50 |
| Dioctylbutamidotriazon | 1,00 |
| Octocrylen | 6,00 |
| Tinosorb® S | 2,00 |
| Aerosil ® R 972 | 0,50 |
| Konservierung | 0,50 |
| Glycerin | 10,00 |
| MgSO₄ | 1,00 |
| Wasser | ad 100,00 |

### Beispiel 5

| W/O - Emulsion | Gew.-% |
|---|---|
| Cetyldimethiconcopolyol | 5,00 |
| Dimethicon | 5,00 |
| Mineralöl | 2,00 |
| Isohexadekan | 2,00 |
| C₁₂₋₁₅-Alkylbenzoat | 5,00 |
| Octocrylen | 15,00 |
| Tinosorb® S | 6,00 |
| Methylbenzylidencampher | 4,00 |
| Butylmethoxydibenzoylmethan | 2,00 |
| Titandioxid | 2,00 |
| Konservierung | 0,50 |
| Glycerin | 5,00 |
| NaCl | 1,00 |
| Phenylbenzimidazolsulfonsäure | 4,00 |
| Natronlauge 45% | 1,30 |
| Wasser | ad 100,00 |

### Beispiel 6

| Hydrodispersion | Gew.-% |
|---|---|
| Caprylsäure/Caprinsäuretriglycerid | 10,00 |
| Octyldodecanol | 5,00 |
| Dicaprylylether | 2,00 |
| Dimethicon | 1,00 |
| Vitamin E-Acetat | 0,50 |
| Octyltriazon | 2,00 |
| Methylbenzylidencampher | 4,00 |
| Butylmethoxydibenzoylmethan | 2,00 |
| Titandioxid | 1,00 |
| Konservierung | 0,50 |
| Glycerin | 3,00 |
| Xanthan Gummi | 0,40 |
| Pemulen ® TR1 | 0,40 |
| Natronlauge 45% | 0,40 |
| Wasser | ad 100,00 |

### Beispiel 7

| W/O - Pickeringemulsion | Gew.-% |
|---|---|
| Caprylsäure/Caprnsäuretriglycerid | 15,00 |
| Hydriertes Polyisobuten | 5,00 |
| C₁₂₋₁₅-Alkylbenzoat | 5,00 |
| Octocrylen | 10,00 |
| Tinosorb® S | 4,00 |
| Titandioxid | 4,00 |
| Aerosil ® R 972 | 2,00 |
| Konservierung | 0,50 |
| Glycerin | 5,00 |
| NaCl | 1,00 |
| Phenylbenzimidazolsulfonsäure | 1,00 |
| Natronlauge 45% | 0,40 |
| Wasser | ad100,00 |

### Beispiel 8

| Spray | Gew.-% |
|---|---|
| Ceteareth-12 | 1,50 |
| Caprylsäure/Caprnsäuretriglycerid | 2,00 |
| Mineralöl | 5,00 |
| Octocrylen | 6,00 |
| Tinosorb® S | 3,00 |
| Octyltriazon | 1 ,00 |
| Konservierung | 0,50 |
| Glycerin | 10,00 |
| Phenylbenzimidazolsulfonsäure | 1,00 |
| Natronlauge 45% | 0,40 |
| Wasser | ad 100,00 |

### Beispiel 9

| Spray | Gew.-% |
|---|---|
| Glycerinmonostearat SE | 4,50 |
| Ceteareth-20 | 1,00 |
| Dicaprylylether | 5,00 |
| Cetylstearylisononanoat | 5,00 |
| Dimethicon | 2,00 |
| Octocrylen | 8,00 |
| Tinosorb® S | 4,00 |
| Konservierung | 0,50 |
| Glycerin | 5,00 |
| Wasser | ad 100,00 |

## Patentansprüche

1. Kosmetische oder dermatologische Lichtschutzzubereitungen mit einem Gehalt an α-Bornitrid und Ethylhexyl-2-cyano-3,3-diphenylacrylat, **dadurch gekennzeichnet, daß** sie 0,1 bis 20 Gew.-% an α-Bornitrid und 0,1 bis 20 Gew.-% an Ethylhexyl-2-cyano-3,3-diphenylacrylat enthalten, wobei die folgende Zusammensetzung (Angaben in Gew.-%) vom Schutzbereich ausgenommen ist:
| | |
|---|---|
| Polyglyceryl-2 Dipolyhydroxystearat | 2 |
| PEG-30 Dipolyhydroxystearat | 2 |
| Caprylic/Capric Triglycerid | 5 |
| Dimethicon | 2 |
| Butylen Glycol Dicaprylat/Caprat | 6 |
| C₁₂₋₁₅- Alkylbenzoat | 6 |
| Vitamen E-Acetat | 0,5 |
| Octyltriazon | 3 |
| Dioctyl Butamido Triazon | 2 |
| Methylbenzyliden Campher | 4 |
| Octocrylen | 10 |
| Bornitrid | 1 |
| Konservierung | 0,5 |
| Glycerin | 5 |
| MgSO₄ | 1 |
| Wasser | ad 100 |

2. Kosmetische oder dermatologische Lichtschutzzubereitungen nach Anspruch 1, **dadurch gekennzeichnet, daß** sie 0,5 bis 10 Gew.-% an α-Bornitrid und 0,5 bis 10 Gew.-% an Ethylhexyl-2-cyano-3,3-diphenylacrylat enthalten.

3. Kosmetische oder dermatologische Lichtschutzzubereitungen nach Anspruch 1, **dadurch gekennzeichnet, daß** sie 1 bis 5 Gew.-% an α-Bornitrid und 1 bis 5 Gew.-% an Ethylhexyl-2-cyano-3,3-diphenylacrylat enthalten.

4. Verwendung von Bornitrid zur Steigerung des Lichtschutzfaktors und/oder der UV-A-Schutzleistung kosmetischer oder dermatologischer Lichtschutzzubereitungen, welche 0,1 bis 20 Gew.-% an Bornitrid und 0,1 bis 20 Gew.-% an Ethylhexyl-2-cyano-3,3-diphenylacrylat enthalten.

5. Verwendung von Bornitrid zur Steigerung des Lichtschutzfaktors und/oder der UV-A-Schutzleistung kosmetischer oder dermatologischer Lichtschutzzubereitungen, welche 0,5 bis 10 Gew.-% an Bornitrid und 0,5 bis 10 Gew.-% an Ethylhexyl-2-cyano-3,3-diphenylacrylat enthalten.

6. Verwendung von Bornitrid zur Steigerung des Lichtschutzfaktors und/oder der UV-A-Schutzleistung kosmetischer oder dermatologischer Lichtschutzzubereitungen, welche 1 bis 5 Gew.-% an Bornitrid und 1 bis 5 Gew.-% an Ethylhexyl-2-cyano-3,3-diphenylacrylat enthalten.

## Claims

1. Cosmetic or dermatological sunscreen formulations with a content of α-boron nitride and ethyl hexyl-2-cyano-3,3-diphenyl acrylate, **characterized in** such way that they contain 0.1 to 20 % by weight α-boron nitride and 0.1 to 20 % by weight ethylhexyl-2-cyano-3.3-diphenyl acrylate, whereby the following composition (data in % by weight) is excluded from the range of protection:
| | |
|---|---|
| Polyglyceryl-2 dipolyhydroxystearate 2 | |
| PEG-30 dipolyhydroxystearate | 2 |
| Caprylic/Capric triglyceride | 5 |
| Dimethicone | 2 |
| Butylene glycol dicaprylate/caprate | 6 |
| C12-15 alkyl benzoate | 6 |
| Vitamin E acetate | 0.5 |
| Octyl triazone | 3 |
| Dioctyl butamido triazone | 2 |
| Methylbenzylidene camphor | 4 |
| Octocrylene | 10 |
| Boron nitride | 1 |
| Preservative | 0.5 |
| Glycerol | 5 |
| MgSo4 | 1 |
| Water | ad 100 |

2. Cosmetic or dermatological sunscreen formulations according to claim 1, **characterized** such that they contain 0.5 to 10% by weight α-boron nitride and 0.5 to 10% by weight ethylhexyl-2-cyano-3.3-diphenyl acrylate.

3. Cosmetic or dermatological sunscreen formulations according to claim 1, **characterized** such that they contain 1 to 5% by weight α-boron nitride and 1 to 5 % by weight ethylhexyl-2-cyano-3.3-diphenyl acrylate.

4. Use of born nitride for potentiation of the sunscreen factor and/or the degree of protection against UV-A of cosmetic or dermatological sunscreen formulations containing 0.1 to 20% by weight boron nitride and 0.1 to 20% by weight ethyl hexyl-2-cyano-3.3-diphenyl acrylate.

5. Use of born nitride for potentiation of the sunscreen factor and/or the degree of protection against UV-A of cosmetic or dermatological sunscreen formulations containing 0.5 to 10 % by weight boron nitride and 0.5 to 10 % by weight ethylhexyl-2-cyano-3.3-diphenyl acrylate.

6. Use of born nitride for potentiation of the sunscreen factor and/or the degree of protection against UV-A of cosmetic or dermatological sunscreen formulations containing 1 to 5% by weight boron nitride and 1 to 5% by weight ethylhexyl-2-cyano-3.3-diphenyl acrylate.

## Revendications

1. Les préparations cosmétiques ou dermatologiques pour protection contre la lumière avec une teneur en Bomitride et en héxyle d'éther 2 de cyanoacrylate de 3,3 acrylate de diphénile **caractérisées par** leur composition de 0,1 à 20 pourcentage en poids en Bomitride et 0,1 à 20 en pourcentage en poids en héxyle d'éther 2 de cyano acrylate et de 3,3 d'Acrylate de diphényle, bien que la composition suivante (données en pourcentage en poids) soit exclue du périmètre de protection.
| | |
|---|---|
| Polyglycéryle 2 Dipolydroxystéarate | 2 |
| PEG-30 Dipolydroxystéarate | 2 |
| Caprylique / Caprique Triglycéride | 5 |
| Diméthicon | 2 |
| Butylène Glycol Dicaprylat / Caprat | 6 |
| C₁₂₋₁₅Alkylbenzoate | 6 |
| Vitamines E Acétate | 0.5 |
| Triazone d'octyle | 3 |
| Dioctyle Butamide Triazone | 2 |
| Camphre de méthylbenzylidène | 4 |
| Octocrylène | 10 |
| Bomitride | 1 |
| Produits de conservation | 0.5 |
| Glycérine | 5 |
| MgSO₄ | 1 |
| Eau | ad 100 |

2. Les préparations cosmétiques ou dermatologiques pour protection contre la lumière aux termes de la revendication 1 **caractérisées par le fait qu'**elles contiennent de 0,5% à 10 en pourcentage de poids en Bomitride et de 0,5 jusqu'à 10 en pourcentage de poids en héxyle d'ether 2 de cyano acrylate et 3,3 d'acrylate de diphényle.

3. Les préparations cosmétiques ou dermatologiques pour protection contre la lumière aux termes de la revendication 1 **caractérisées par le fait qu'**elles contiennent de 1 à 5 en pourcentage de poids en Bomitride et de 1 jusqu'à 5 en pourcentage de poids en héxyle d'éther, 2 de cyano acrylate et 3,3 d'acrylate de diphényle.

4. L'utilisation de Bomitride pour augmenter le fateur de protection contre la lumière ou la capacité de protection contre les rayons ultraviolets des préparations cosmétiques ou dermatologiques pour protection contre la lumière ou les deux à la fois, lesquelles contiennent de 0,1 jusqu'à 20 en pourcentage de poids de Bomitride et de 0,1 jusqu'à 20 en pourcentage de poids en héxyle d'éther, 2 de cyano acrylate et 3,3 d'acrylate de diphényle.

5. L'utilisation de Bomitride pour augmenter le fateur de protection contre la lumière ou la capacité de protection contre les rayons ultraviolets des préparations cosmétiques ou dermatologiques pour protection contre la lumière ou les deux à la fois, lesquelles contiennent de 0,5 jusqu'à 10 en pourcentage de poids de Bomitride et de 0,5 jusqu'à 10 en pourcentage de poids en héxyle d'éther, 2 de cyano acrylate et 3,3 d'acrylate de diphényle.

6. L'utilisation de Bomitride pour augmenter le fateur de protection contre la lumière ou la capacité de protection contre les rayons ultraviolets des préparations cosmétiques ou dermatologiques pour protection contre la lumière ou les deux à la fois, lesquelles contiennent de 1 jusqu'à 5 en pourcentage de poids de Bomitride et de 1 jusqu'à 5 en pourcentage de poids en héxyle d'éther, 2 de cyano acrylate et 3,3 d'acrylate de diphényle
